# EUROPEAN PATENT SPECIFICATION

(11) **EP 1 228 757 B1**
(45) Date of publication and mention of the grant of the patent: **15.12.2004**
(21) Application number: 01600015.0
(22) Date of filing: 05.06.2001
(51) Int. Cl.: A61K 31/18, A61K 47/22, A61K 47/10

(54) **Stable pharmaceutical solutions of nimesulide**
Stabile pharmazeutische Nimesulidverbindungen
Solution pharmaceutique stable de nimésulide

(30) Priority: 05.02.2001 GR 2001100050
(43) Date of publication of application: 07.08.2002
(73) Proprietor: Uni-Pharma Kleon Tsetis Pharmaceutical Laboratories S.A., 145 64 Kifissia (GR)
(72) Inventor: Tsetis, Kleon, 145 61 Kifissia (GR); Tsetis, Anastasios, 104 34 Athens (GR); Tsetis, Julia, 145 64 Kifissia (GR)

(56) References cited:
- EP-A- 0 146 414
- EP-A- 0 916 347
- EP-A- 1 002 531
- WO-A-00/48588

## Description

### Background of the invention

The present invention relates to stable pharmaceutical solutions, containing Nimesulide as active substance, fabricated for the first time and suitable for parenteral administration.

Nimesulide, N-(4-nitro-2-phenoxy-methan-sulfanilide, is a potent non-steroidal anti-inflammatory (NSAID's) drug, presently used in the treatment of painful inflammatory conditions, due to rheumatoid arthritis, which also possesses antipyretic activity.

Compared to other NSAIDs, Nimesulide has a better therapeutic ratio, low gastrotoxicity and generally good tolerability.

Nimesulide is a strongly hydrophobic substance that is practically insoluble in water (solubility in water at room temperature: 0,01mg/ml).

Its disadvantages, as being insoluble in water and to a large number of non-toxic solvents as well, illustrate the inability of preparing until today, solutions of Nimesulide, useful for therapeutic purposes under the form of solutions suitable for intramuscular or intravenous use.

It is known and well accepted that parenteral administration of drugs for the treatment of painful inflammatory conditions, is more effective than other routes of administration, since the drug enters the circulation directly and promptly manifests its therapeutic effect.

EP1002351 describes Injectable solutions of Nimesulide using dimethylacetamide as solubilization agent.

WO0048588 discloses topical formulations of nimesulide containing glycerol formal in emulsions

EP0146414 describes glycerol formal used to solubilise avermectin and milbermycin

Rep0916347 discloses the use of glycerol formal used to solubilise paracetamol

The aim of the present invention was to overcome the disadvantages of Nimesulide's solubility into solvents considered to be suitable for injectable solutions of drugs and to also obtain one or more solutions of Nimesulide safe and harmless during parenteral administration under the form of several pharmaceutical preparations like suppositories and ointments.

The inventors of the present invention have conducted extended experiments using a large scale of non-toxic solvents among which Glycerin formaldeyde, Propylene glycol and Ethanol and discovered that their combination in various percentage rates consist a dissolver system into which Nimesulide is promptly solved producing a clear solution of pale yellow color, which due to the very low toxicity of its components, is suitable for parenteral administration.

Glycerol formal when administered endo-peritoneally shows very low toxicity (LD₅₀ - 3000mg/kg to albino rats) therefore, it is a very well tested and admissible solvent for the preparation of parenterally administered solutions.

Propylene glycol is also a very suitable and acceptable solvent for pharmaceutical substances deprived of toxic side effects, since its LD₅₀ is higher than 8000mg/kg when it is endo-peritoneally administered to albino rats. (D.M. ANDERSON: J.Pharm. Pharmacol., 1959, 11, 150).

According to the present invention it is the first time that Nimesulide solutions can be prepared based on hydro-organic system, consisting of Glycerol formal, Ethanol and Water, the composition of which by volume stands thus :

| | |
|---|---|
| Glycerol formal | 80,0ml |
| Ethanol | 15,0ml |
| Water q.s. to | 100 ml |

There is also another hydro-organic system into which Nimesulide is easy solved, which is made up of Glycerol formal, Propylene glycol, Ethanol and Water, where the content by volume of Glycerol formal is 75% v/v, of Ethanol 10% v/v, of Propylene glycol 10% v/v and water 5% v/v.

To the above described hydro-organic solvent system, Nimesulide is easily solved in several contents producing solutions where the concentration of Nimesulide ranges from 10 to 30mg/ml, giving thus the opportunity for the preparation of Nimesulide's solutions containing 50, 75, 100, 150 and 200mg therapeutical doses.

These Nimesulide's solutions are clear, odorless, pale-yellow coloured, very stable, almost non-toxic, therefore, quite suitable for parenteral administration of Nimesulide for therapeutic purposes. Furthermore, they may be improved by the addition of antioxidants, local anaesthetics, buffers and chelating agents as well.

Another aim of the present invention is to reveal the method by which these solutions are made up and this is described bellow.

### Method of preparation

To the slightly pre-heated Glycerol formal is added, under continuous stirring, the required and desirable quantity of Nimesulide, until a clear pale-yellow solution is obtained. After cooling at room temperature Ethanol or Propylene glycol, according to the case, are added plus the required water as well, which contains the above prescribed auxilliary agents.

Filtration is followed and the obtained clear solutions of Nimesulide are suitable for therapeutic purposes when administered under various pharmaceutical formulations like ointments, creams, suppositories and particularly, due to their very low toxicity, in the form of injections for intramuscular or intravenous use.

## Claims

1. Stable pharmaceutical solutions of Nimesulide in a hydro-organic solvent system, consisting of 80% vlv Glycerol formal, 15% v/v Ethanol and 5% v/v Water.

2. Stable pharmaceutical solutions of Nimesulide in a hydro-organic solvent system, consisting of 75% v/v Glycerol formal, 10% v/v Ethanol, 10% v/v Propylene glycol and 5% Water v/v.

3. Stable pharmaceutical solutions of Nimesulide, according to claims 1 and 2 containing Nimesulide in concentrations of 10 to 30mg/ml, suitable for the preparation of pharmaceutical forms of parenteral administration destined to therapeutic purposes.

4. Stable pharmaceutical solutions according to claims 1, 2 and 3, which can be improved by the addition of auxilliary substances, like antioxidants, chelating agents, surfactants, local anaesthetics, preservatives.

## Patentansprüche

1. Stabile pharmazeutische Nimesulid Lösungen, welche aus 80% glycerino-Formaldehyd, 15% Ethanol und 5% Wasser zusammengesetzt werden.

2. Stabile pharmazeutische Nimesulid Lösungen, welche aus 75% glycerino-Formaldehyd, 10% Ethanol, 10% Propylenoglycol und 5% Wasser zusammengesetzt werden.

3. Stabile pharmazeutische Nimesulid Lösungen, nach Anspruchen 1 und 2, welche Nimesulid in einer Lösungskonzentration von 10-30mg/ml enthalten und geeignet sind für die Herstellung pharmazeutischer Produkte, parenteraler Verabreichung für therapeutische Zwecke.

4. Stabile pharmazeutische Nimesulid Lösungen, nach Ansprüchen 1 und 2 und 3 denen Hilfsstoffe zugesetzt werden können, wie antioxydative und chelose Faktoren, Detergenzien, lokale anesthetika, konservierungsmittel.

## Revendications

1. Stables solutions pharmaceutiques de Nimesulide dans un système aqueux-organique constitue de 80% v/v Glycerol Formal, 15% v/v Ethanol et 5% v/v d' Eau.

2. Stables solutions pharmaceutiques de Nimesulide dans un système aqueux-organique constitue de 75% v/v Glycerol Formal, 10% v/v Ethanol et 5% v/v d' Eau.

3. Stables solutions pharmaceutiques de Nimesulide qui sont en accord avec les revendications 1 et 2 contenant Nimesulide au concentrations des 10-30mg/ml convenable pour la préparation de formulations pharmaceutiques injectables destinées au pursuites therapeutiques.

4. Stables solutions pharmaceutiques de Nimesulide qui sont en accord avec les revendications 1, 2 et 3 qui peuvent etre améliorer avec l'addition de substances auxiliaires comme par example d'antioxidants, agents de chelation, surfactants, anesthetiques locales, préservatifs.
